# EUROPEAN PATENT SPECIFICATION

(11) **EP 2 812 009 B1**
(45) Date of publication and mention of the grant of the patent: **30.05.2018**
(21) Application number: 13715452.2
(22) Date of filing: 07.02.2013
(51) Int. Cl.: A61K 31/722, A61K 31/736, A61K 31/12, A61K 33/06, A61K 33/10, A61K 36/888, A61K 36/9066, A61P 1/04

(54) **PRODUCT COMPRISING GLUCOMANNAN AND CHITOSAN FOR THE TREATMENT OF GASTROESOPHAGEAL REFLUX DISEASE**
PRODUKT MIT GLUCOMANNAN UND CHITOSAN ZUR BEHANDLUNG VON REFLUXÖSOPHAGITIS
PRODUIT COMPRENANT DU GLUCOMANNANE ET DU CHITOSANE POUR LE TRAITEMENT DES MALADIES DE REFLUX GASTRO-OESOPHAGIEN

(30) Priority: 08.02.2012 IT RM20120004
(43) Date of publication of application: 17.12.2014
(73) Proprietor: Dicofarm Spa, 00138 Roma (IT)
(72) Inventor: MARTELLI, Laura, 00138 Roma (IT); MARTELLI, Mario, 00138 Roma (IT); FERRACCHIATTI, Piero, 00138 Roma (IT)
(74) Representative: Modiano, Micaela Nadia
(86) International application number: PCT/IT2013/000039
(87) International publication number: WO 2013/121452

(56) References cited:
- EP-A1- 1 859 786
- GB-A- 2 327 074

## Description

In its general aspect, the present invention relates to nutraceuticals, dietary supplements and medical device products based on an association of glucomannan, chitosan and carbonates to prevent or treat the condition of gastroesophageal reflux.

Gastroesophageal reflux is a common symptom characterized by abnormal rise of gastric acid into the esophagus. Normally the ingested food, as a result of swallowing, passes through the esophagus, which leads the food bolus into the stomach; here the strongly acidic medium allows the digestion of foods, whose absorption occurs in the intestine.

Normally in the esophagus peristaltic waves and the saliva itself operate to allow the progression of the ingested food and material from the esophagus to the stomach. In normal subjects there are defense mechanisms preventing the reflux of acid into the esophagus.

However, approximately 2% healthy individuals may experience regurgitation even standing.

When gastroesophageal reflux, exceeding the normal defense mechanisms of the esophageal mucosa, from physiological condition becomes pathological, an opposite movement of food and stomach acid occurs from the stomach into the esophagus. Esophagus is not provided with protection systems against the hydrochloric acid produced by the stomach, therefore the irritation of esophagus epithelium causes retrosternal burning sensation, painful swallowing and acid belching.

Gastroesophageal reflux is very common, sometimes it is temporary, sometimes is symptomatic of a more serious pathology.

In less severe cases the treatment of gastroesophageal reflux disease is usually based on some basic dietary and hygiene standards. Proper diet and appropriate lifestyle can greatly help the healing process.

The pharmacological treatment of severe cases is based on proton pump inhibitors (PPI) drugs intake, for differently prolonged periods, significantly inhibiting acid production in the stomach, e.g., omeprazole, lansoprazole, esomeprazole; H2-receptor antagonist (however, largely replaced by the most recent and powerful IPP), for example, cimetidine, ranitidine, famotidine, nizatidine, roxatidina, which reduce gastric acid secretion and, consequently, the reflux; antacids agents, e.g., bicarbonate, magnesium hydroxide and aluminum hydroxide; prokinetic agents, e.g., metoclopramide, domperidone, levosulpiride, promoting gastric emptying; cytoprotective agents, e.g. sucralfate that creates a protective layer preventing the hydrochloric acid action on the gastric mucosa, alginates, natural compounds extracted from marine algae that act by creating a mechanical barrier to reflux.

Only in extreme circumstances surgery is indicated in the treatment of gastroesophageal reflux disease, whenever the cause of reflux is structural and depends on a particular conformation or dysfunction of the involved organs, in particular the cardias, that is removed or surgically corrected.

Therefore, the choice of drug treatment useful for gastroesophageal reflux is very broad and many treatment protocols are available. Furthermore, there are no clear indications to prefer one type of treatment over another, hence, with the exception of surgery, often the physician chooses on the basis of personal experience before being able to identify the type of treatment which best suites to the patient.

Usually for patients with mild or occasional symptoms, treatment with antacid agents, alginates and prokinetic is preferred; in more severe cases proton pump inhibitors and blockers of H2 receptor are recommended.

The use of proton pump inhibitors (PPIs) is widespread, they are drugs generally well tolerated. Most side effects, such as diarrhea, headache, flatulence, abdominal pain, lightheadedness/dizziness, rashes, heart palpitations are mild and transient, disappear when the treatment is stopped. However, following prolonged use of PPI an increased incidence of osteoporosis was found, with a considerable increase in the number of fractures of the neck of the femur resulting from the resorption of calcium due to induction of hypochlorhydria.

The basic salts of aluminum, magnesium and calcium, taken by mouth, exert their antacid action locally in the esophagus and then are eliminated, providing a very fast benefit, but of short duration. In case of excessive or prolonged intake, side effects can appear. These active ingredients may affect the absorption of other drugs, interfering with any other therapies in place.

Cytoprotectors are used in the treatment of gastroesophageal reflux for their ability to protect the stomach mucosa by forming a barrier against gastric acid juices thereon. Even a possible ulcerative lesion, if already formed, is protected and can more easily heal. Although this class of drugs is generally well tolerated, however, in addition to the beneficial effects it is possible to experience some side effects; frequently disorders such as constipation, gastrointestinal disturbances, dry mouth, itching and skin disorders may appear, primarily due to the presence of aluminum salts in the chemical structure of this kind of drug.

Drugs containing alginate as active ingredient do not have a proper pharmacological action, but physically block the reflux. When alginate reaches the stomach comes into contact with the gastric acids and forms a gel that floats on top of the stomach contents. This gel acts as antireflux barrier, positioned in the stomach prevents acid material rising into the esophagus. Alginates, in form of tablets or liquid suspension, act rapidly, are not digested by gastrointestinal enzymes, do not interfere with the digestion and can also be employed together with other drugs; however, they have the unfavorable effect of promoting fermentation in the colon, which creates considerable discomfort to the patient.

In view of the large and growing spread of the disease, in Italy the disorder is very common, affecting about one out three subjects, and side effects related to the treatment duration, it is still strongly desired the development of alternative therapeutic products for the use in the prevention and treatment of symptoms related to gastroesophageal reflux which avoid the patient of incurring side effects for the prolonged administration of main antacids currently available and used.

Moreover, if the reduction of the amount of gastric acid is the first target in the treatment of symptoms of gastroesophageal reflux, it should be noted that neutralization, or total elimination, of H⁺ ions presents serious consequences for the organism, such as increase of serum gastrin, possibility of producing nitrosamines and pro-cancerogens formation. In order to be really safe and effective, an antacid agent should be able to counteract the acidity in the stomach, without to raise the pH value avoiding the so-called "acid rebound". Accordingly, the best therapeutic approach, providing greater benefit to the patient, is, not to lower the H⁺ ions concentration, but to modulate keeping the H⁺ ions at a such concentration not to create discomfort.

In the prior art, a composition enabling modular gastric concentration of H⁺, further providing a physical barrier to gastroesophageal reflux, modular itself in the use for the treatment of gastroesophageal reflux, providing to the recipient of the treatment the possibility of choose the viscosity degree of the preparation according to its acceptance and necessity is not described.

In fact, so far in pharmaceuticals forming a physical barrier to the rising of gastric acid into the esophagus, such as Gaviscon™ and the like, the raising of the pH is delegated to the use of carbonates and bicarbonates of alkali and alkaline earth elements, whereas the viscosity is determined by the type of alginate chosen (ref. U.S. 4,140,760).

The international patent application WO2010/081720 describes oral compositions based on polysaccharides derived from cladodes of Opuntia Ficus Indica in combination with active substances for prevention and treatment of gastroesophageal reflux disease and related diseases. Such active substances are those typically used to treat reflux and widely described above: antacid agents, proton pump inhibitors, bicarbonates, prokinetic agents, alginic acid or alginates, chitosan. The polysaccharides derived from Opuntia Ficus Indica provide a sort of mucilage with mucoadhesive capacity which retains various active substances and acts according to various mechanisms at gastric mucosa level.

The international patent application WO99/63986, in the name of Reckitt & Colman Products Limited, describes formulations for the treatment of gastroesophageal reflux disease comprising a pharmaceutical carrier forming a floating barrier layer or a bioadhesive layer that precedes gastric acid in contact with the mucosa and protects it, and an active ingredient selected in the group of capsaicin and other structurally related spicy compounds such as zingerone, curcumin, piperine, resiniferatoxin. According to this invention the active ingredient, able to promote the healing of gastric ulcers experimentally induced, is orally administered in a product containing alginate. Example 9 describes a soluble chitosan coating the active product.

Another invention of Reckitt & Colman Products Limited described in WO00/67799 teaches how to improve the bioadhesive properties of formulations for the treatment of disorders of the esophagus using a combination of a) alginate, b) a rubber selected in the group of xanthan gum, carrageenan and mixtures thereof, and c) glucomannan or galactomannan. Mixtures of a gum and glucomannan or galactomannan are widely used as thickening and gelling agents in the food industry, wherein glucomannan or galactomannan in association with alginate confers a such viscosity level to ensure improved adhesive properties, and consequently protective, in particular on the esophageal mucosa. However, it should be noted that a very thick product does not receive the acceptance of a large part of the public, because of its poor pleasantness to taste and to sight.

The patent application WO2010/031785 provides a solution to such technical problem of poor palatability of pharmaceutical compositions for the treatment of gastroesophageal reflux disease by using phospholipids in combination with active ingredients, such as sucralfate, to be administered orally. The compositions according to this invention may also include additional substances with antacid activity such as aluminum hydroxide, magnesium hydroxide, carbonate or bicarbonate, simethicone, salts of alginic acid, hydrolysed and non-hydrolysed chitins, saccharide polymers such as glucomannans or PEG, misoprostol and proton pump inhibitors, or active ingredients increasing the esophageal sphincter tone and accelerate gastric emptying as well. The application GB2327074 discloses microcapsules effective in the treatment of disorders related to gastroesophageal reflux disease (GERD) composed by alginate and chitosan and comprising calcium carbonate as the active ingredient. The product has the advantage that adheres to the affected site providing local acid neutralization and relief from heartburn caused by acid reflux on the esophageal mucosa.
EP1859786 discloses a preparation for reducing the symptoms of heartburn and gastroesophageal reflux disease, comprising polysaccharides forming a highly viscous protective layer in the stomach. Preferred polymers are galactomannans and glucomannans. Chitosans and carbonates are also mentioned as possible ingredients. The present invention is aimed to provide a preparation able to modulate, and not to abolish, the gastric concentration of H⁺ ions and provide a physical barrier to reflux, modular itself, allowing the patient recipient of the treatment to choose and determine the viscosity degree of the preparation according to the severity degree of the symptoms and their acceptance.

Surprisingly, the inventors have found that using an organic base it is possible to modulate the amount of H⁺ ions in the stomach, and that the association of glucomannan with such organic base provides a product to prepare extemporaneous solutions/suspensions acting as barrier to reflux allowing the final consumers to choose and determine the level of viscosity according to their needs and acceptability.

Such result cannot be obtained by using alginates, or at least alginates alone, because these easily form lumps during their extemporaneous use.

A further aim of the invention is to prolong the controlling time of the gastric pH by the composition by integrating the association of glucomannan and chitosan with one ore more carbonates. In such a way the invention achieves the control of gastric contents by modulation into two sequential phases, the first due to carbonates action and the second due to chitosan. Infact, chitosan has a pKb in the range from 6 to 10 able to form weak acids in acidic environment maintaining a residual acidity which is fundamental for the health.

Constitutes a further aim of the invention to provide in combination with the composition comprising chitosan, glucomannan and carbonates the use of powder of *Curcuma longa* and/or curcumin which promotes the gastroesophageal sphincter closure by inhibiting the NO synthase.

It is therefore a first aspect of the disclosure a nutraceutical preparation and/or food supplement and/or medical device for the use in the treatment of gastric reflux comprising the association of glucomannan, or its polymers and/or blends of polymers derived therefrom, and an organic base, i.e. chitosan, or its polymers and/or blends of polymers derivatives thereof, with a pKb in the range from 6 to 10, such that in the presence of H⁺ ions yields weak conjugate acids with pKa ranging from 8 to 4, being such association adapted to modulate the acidity level of the gastric contents which in turn is blocked in the rising to the esophagus.

A second aspect of the disclosure is a nutraceutical preparation and/or food supplement and/or medical device product for the use in the treatment of gastric reflux comprising the association of glucomannan and an organic base, i.e. chitosan, and one or more carbonates. An embodiment of the invention is a nutraceutical preparation and/or food supplement and/or medical device product for the use in the treatment of gastric reflux comprising the association of glucomannan, chitosan, one or more carbonates, and curcumin and/or *Curcuma longa* powder. Chitosan is a linear polysaccharide composed of D-glucosamine and N-acetyl-D-glucosamine linked by β bonds (1-4), obtained by alkaline deacetylation of natural chitin.

The molecule of chitosan is insoluble in water, but is soluble in acid solution, wherein its free amino groups are protonated, and the greater the amount of these charged groups, the higher the viscosity of the polyelectrolyte.

Chitosan solubilizes in contact with the gastric juices forming amine groups with positive electrostatic charge able to bind the negative carboxyl groups of free fatty acids and bile acids and to interact with gastric mucin.

Chitosan has a wide use in the formulation of products for ophthalmic, nasal, oral application and, in general, modified-release, thanks to its properties of muco-adhesiveness and drug carriers.

It is known that chitosan is not absorbed in the gastrointestinal tract (Murata Y. et al. 2003. Biol. Pharm. Bull. 36, 687-90).

The inventors have observed that when 0.1 g of chitosan is added to distilled water (20ml) under stirring, the solid form of chitosan is dispersed but does not solubilize. After about 30 minutes the pH value detected is equal to 9.21±0.167 (average of three measurements). When 1.5 g of chitosan are added to 50ml of 0.1 N HCl solution and the dispersion is maintained under stirring for 12 hours at room temperature, the resulting solution becomes very viscous and shows the presence of residual suspended solid particles, while the pH value of the suspension is about 5.0 and such is maintained while continuing to add HCl until all the chitosan dissolves.

The titration of the chitosan solution in HCl shows that -NH₃⁺ group has a pKa equal to 5, corresponding to a weak acid. This observation allows to assume that in the gastric environment chitosan is able to drain the H⁺ ions progressively until free amino groups are available, allowing, however, the persistence in the medium of a residual amount of H⁺ due to the equilibrium constant of the conjugate acid.

Glucomannan, in association with chitosan in the composition according to the invention, is a water-soluble high molecular weight polysaccharide of units of D-mannose and D-glucose, linked by β bonds (1-4). Glucomannan is a water-soluble granular powder, characterized by high viscosity and ability to swell by water absorption up to 60 times its weight. This property is maintained in an acidic environment, that's why in the stomach, glucomannan, turning into jelly, gives a satiety feeling; for this feature it is widely used as a food additive, emulsifier and thickener.

The molecular weight of the glucomannan used in the present invention preferably is in the range between 1.9 x10⁶ and 1x10⁴.

The glucomannan of the present invention is naturally or by a synthesis process derived, but preferably is derived from a natural source, in particular from *Amorphophallus konjac* root, a perennial herbaceous plant. Almost two-thirds of dry matter of its tuber contains glucomannan. In the molecule of glucomannan of the association according to the invention mannose and glucose are respectively present in a ratio of 1.6:1.

It has been observed that due to the addition of 1.5 g of glucomannan in 50ml of 0.1 N HC1, the formation of a thick gel, which tends to incorporate all the solid residues present in the solution, gradually occurs. Hence, in the gastric environment glucomannan, became more viscous even in the presence of acids, can immobilize the stomach contents preventing the same may leak back into the esophagus.

The presence of chitosan ensures that the buffering action on gastric hyperacidity is maintained over time around a value equal to 5 prolonging the beneficial effect for the patient.

In order to prolong the pH control time, in a particularly preferred embodiment of the invention the active ingredient is constituted by the association of glucomannan, chitosan and carbonates. The carbonates presence determines the buffering response to the high gastric acidity is rapidly established, which, however, is stabilized and maintained around values close to 5 by the organic base action. Hence, the composition is characterized in being able to modulate the pH of gastric contents into two sequential phases, the first due to the action of the carbonates present in the association constituting the active ingredient and the second due to the chitosan.

In a preferred embodiment of the invention the composition is in the form of solid powder, suitable to be orally administered by the recipient of the treatment. The patient dissolving the solid powder in a proper solvent can define the level of viscosity according to his/her degree of acceptance. Once in the stomach, the carbonates of the composition interact with the gastric acid and neutralize them, which leads to a rapid rise in the pH value. When all carbonates have reacted, and the pH tends to lower (rebound effect), the action of the organic base, chitosan, intervenes, in the gastric acid it is protonated becoming the relative conjugate acid. This stabilizes the pH value around 5, determining a modulation of the pH value in two phases, and for a prolonged time, and not a rapid and abrupt turning of its value, and any case, of short duration.

The carbonates in the association of the present invention are selected from the group consisting of basic carbonate of magnesium and calcium carbonate.

Moreover, it is noteworthy considering that, the use of the present composition, reducing gastric acidity without canceling it out completely, prevents the increase of serum gastrin, the production of nitrosamines and the formation of procancerogenic agents.

All polymers of chitosan and glucomannan and/or mixtures of polymers derivatives thereof may be further added to the association of the invention.

According to the invention the association of chitosan and glucomannan further comprises powder of *Curcuma longa* and/or curcumin.

Curcumin is the main biologically active component of *Curcuma longa.* The chemical formula of curcumin is C₂₁H₂₀O₆; the substance is also known as diferuloylmethane and the structural formula is as follows:

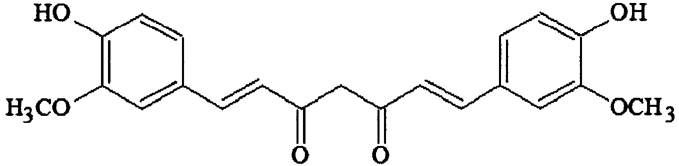

Curcumin, probably best known as the yellow coloring agent in curry, a traditional spice used in cooking and medicine of South-East Asia, has been used for centuries in many Asian cultures. For example, some Indian medical practices use curcumin to treat anorexia, cough, rheumatism, and other diseases. The traditional Chinese medicine uses this compound to treat diseases accompanied by abdominal pain. Recently Western medicine has attributed to curcumin antiinflammatory, anti-oxidant, and anti-bacterial properties, and some positive activity against Alzheimer's disease. Currently several studies and clinical trials are on going to demonstrate the medical properties attributed to curcumin as a natural treatment for several diseases.

The combination according to the present invention may further comprise other substances having gelling properties and/or thickeners, such as, for non limitative example, alginates and its derivatives, polyvinylalcohol, poly(ethyloxazoline), poly(2-hydroxyethylacrylate), pectins, arabic gum, carboxymethylcellulose, mixtures of water-soluble polysaccharides derived from plant fibers marketed under the name of PGX®.

The composition for the suppression of gastroesophageal reflux according to the invention further comprises pharmaceutical acceptable excipients such as solvents, dispersing agents, carriers, phospholipids, flavorings and colorings.

The composition according to the invention is suitable for oral administration and is prepared in solid form, such as powder, granules, capsules, tablets, etc.; in liquid form, such as suspension, solution, emulsion, gel, etc.; in single use sachets for extemporaneous administration. Liquid formulations must be added with suspending agents to maintain suspension of the chitosan and various components.

The composition for the suppression of gastroesophageal reflux comprises chitosan, glucomannan from 0.3 to 10 parts by weight of chitosan, preferably 3.5 parts by weight of chitosan, basic carbonate of magnesium and calcium carbonate, each from 0.1 to 8 parts by weight of chitosan, preferably 0.5 parts by weight of chitosan, powder of *Curcuma longa* and/or curcumin from 0.1 to 16 parts by weight of chitosan, preferably 0.3 parts by weight of chitosan.

Hence the composition containing the association according to the invention is able to exert control:
1) using the glucomannan as viscosifier, but leaving the possibility to the consumer to modulate the viscosity of the extemporaneous solution-suspension preparation according to its palatability;
2) providing a control of gastric pH modulated in two stages, one almost immediate, depending on the carbonates, and a subsequent one which prolongs the time for controlling pH, depending on organic base with a pKb in the range 6 to 10, such as to form a weak acid in an acid environment, able therefore to leave a residual gastric acidity fundamental to the health;
3) using curcumin powder allowing the inhibition of NO synthase, thus promoting the gastroesophageal sphincter closure.

Furthermore, the liberation of CO₂ released into the environment by the acid carbonates promotes the mixing of the viscous solution of the preparation with the foreign bodies of the gastric contents, and the consequent rise in pH favors itself a further rise in viscosity.

Conclusively, the described preparation for the use in the treatment of gastroaesophageal reflux is assumed after meals as a suspension-aqueous solution, is based on the association of glucomannan, curcumin, carbonates and a specific organic base (i.e. chitosan), which is able to keep the pH value about 5, thus avoiding levels that may lead to the formation of nitrosamines. It is remarkable consider that the invention makes use of the innovative application of chitosan to revert and maintain the pH to a value practically buffered equal to 5. Furthermore, this modulation of pH value is established subsequently to an early control exerted by carbonates of the association, thus allowing a widening of the times of gastric pH control, with greater benefits for the patient.

Further characteristics and advantages of the present invention will appear clearly from the following examples.

### EXAMPLE

The following examples illustrate the experimental use in vitro of the invention. It was yielded a preparation comprising:

| | |
|---|---|
| glucomannan | 55 g |
| chitosan | 16 g |
| magnesium basic carbonate | 8 g |
| calcium carbonate | 8 g |
| Curcumin | 6 g |
| liquorice powder | 3 g |
| anise | 2 g |
| sucrose | 2 g |

Three grams of this mixture were added to 50ml of distilled water, the suspension was mixed and after 4-6 minutes was obtained a viscous solution with pH about 8.0.

The obtained viscous solution was added to 500ml of 0.1 N HCl (pH 1.0); several insoluble bodies were floating in the resulting solution, the measurement of pH after the addition showed that the pH value had risen up to about 5-6; while the solution, after an initial drop in viscosity due to dilution, over time tended to be more and more viscous. At this point it was noted that the pH value of the solution remained more or less constant, and foreign bodies insoluble in the solution of HC1, were included-trapped-thickened in the viscous gel.

Following the addition of the preparation to water, during mixing, the viscosity reaches the maximum within 60-90 minutes. Therefore, these features allow the easy mixing of the preparation with any contents of the container wherein the viscous preparation is poured. Subsequently, increasing the viscosity, greater is the inclusion-entrapment of foreign bodies such to make limited or very limited their movements.

The same results were obtained when in the described composition 10g of glucomannan were replaced with 10g of alginic acid.

## Claims

1. Composition for the use in the prevention and the treatment of disorders related to gastroesophageal reflux disease and its complications comprising the association **characterized in that** it comprises chitosan, glucomannan, and powder of *Curcuma longa* and/or curcumin, being such association the active principle of the composition suitable to modulate the acidity rate of the gastric content which is prevented in the rising into the esophagus.

2. Composition for use according to claim 1, **characterized in that** it further comprises one or more carbonates to control the pH of gastric contents into two steps in sequence, the first step being due to action of the carbonates present in the association, the second step due to the action of chitosan.

3. Composition for use according to claims 1 and 2, **characterized in that** it comprises the association of polymers of chitosan and/or mixtures of polymers thereof, and polymers of glucomannan and/or mixtures of polymers thereof.

4. Composition for use according to claim 1 and 2 **characterized in that** chitosan has pKb in the range between 6 and 10, such that in the presence of H⁺ ions it yields weak conjugate acids with pKa ranging from 8 to 4.

5. Composition for use according to claim 1 and 2 **characterized in that** mannose and glucose in glucomannan molecule are respectively in a ratio of 1.6:1.

6. Composition for use according to claims 1 to 5 **characterized in that** the glucomannan has a molecular weight comprised between 1.9 x10⁶ and 1x10⁴.

7. Composition for use according to claims 1 to 6 **characterized in that** the glucomannan is derived from a natural source, preferably from *Amorphophallus konjac* root.

8. Composition for use according to claims 1 to 6 **characterized in that** the glucomannan is obtained by a synthesis process.

9. Composition for use according to claim 2 **characterized in that** the carbonates are selected from the group consisting of magnesium basic carbonate and calcium carbonate.

10. Composition for use according to any of the preceding claims **characterized in that** it further comprises, alone or in combination, alginates and its derivatives, polyvinyl alcohol, poly (ethyloxazoline), poly (2-hydroxyethyl acrylate), pectins, gum arabic, carboxymethylcellulose, mixtures of water-soluble polysaccharides derived from plant fibers marketed under the trade name PGX®.

11. Composition for use according to any of the preceding claims **characterized in that** it further comprises acceptable pharmaceutical excipients, such as solvents, dispersants, carrier, phospholipids, flavorings and colorings.

12. Composition for use according to any of the preceding claims **characterized in that** it is suitable for oral administration.

13. Composition for use according to claim 12 **characterized in that** it is in solid form, as powder, granules, capsules, tablets, in liquid form, as suspension, solution, emulsion, gel, sachets for extemporaneous administration.

14. Composition for use according to any preceding claims **characterized in that** it comprises: chitosan, glucomannan: 0.3 to 10 parts by weight of chitosan, preferably 3.5 parts by weight of chitosan, basic magnesium carbonate: 0.1 to 8 parts by weight of chitosan, preferably 0.5 parts by weight of chitosan, calcium carbonate: from 0.1 to 8 parts by weight of chitosan, preferably 0.5 parts by weight of chitosan, powder of *Curcuma longa* and/or curcumin: from 0.1 to 16 parts by weight of chitosan, preferably 0.3 parts by weight of chitosan.

15. Composition for use according to any one of the preceding claims **characterized in that** it comprises: chitosan, glucomannan: 3.5 parts by weight of chitosan, basic magnesium carbonate: 0.5 parts by weight of chitosan, calcium carbonate: 0.5 parts by weight of chitosan, powder of *Curcuma longa* and/or curcumin: 0.3 parts by weight of chitosan.

## Patentansprüche

1. Zusammensetzung zur Verwendung in der Vorbeugung und Behandlung von Störungen im Zusammenhang mit gastroösophagealem Rückfluss und ihren Komplikationen, umfassend die Verbindung, **dadurch gekennzeichnet, dass** sie Chitosan, Glucomannan und Pulver von *Curcuma longa* und/oder Curcumin umfasst, wobei solche Verbindung das aktive Prinzip der Zusammensetzung ist, die geeignet ist, die Aziditätsrate des Mageninhalts zu modulieren, der am Aufsteigen in die Speiseröhre gehindert wird.

2. Zusammensetzung zur Verwendung gemäß Anspruch 1, **dadurch gekennzeichnet, dass** sie weiter ein oder mehrere Carbonate umfasst, um den pH-Wert des Mageninhalts in zwei aufeinander folgenden Schritten zu steuern, wobei der erste Schritt auf der Wirkung der Carbonate beruht, die in der Verbindung vorhanden sind, und der zweite Schritt auf der Wirkung von Chitosan beruht.

3. Zusammensetzung zur Verwendung gemäß den Ansprüchen 1 und 2, **dadurch gekennzeichnet, dass** sie die Verbindung von Polymeren von Chitosan und/oder Mischungen von Polymeren davon und von Polymeren von Glucomannan und/oder Mischungen von Polymeren davon umfasst.

4. Zusammensetzung zur Verwendung gemäß den Ansprüchen 1 und 2, **dadurch gekennzeichnet, dass** Chitosan einen pKb-Wert im Bereich zwischen 6 und 10 hat, so dass es in Anwesenheit von H⁺-Ionen schwache korrespondierende Säuren mit einem pKa-Wert im Bereich von 8 bis 4 liefert.

5. Zusammensetzung zur Verwendung gemäß den Ansprüchen 1 und 2, **dadurch gekennzeichnet, dass** Mannose und Glucose im Glucomannanmolekül jeweils in einem Verhältnis von 1,6:1 vorliegen.

6. Zusammensetzung zur Verwendung gemäß den Ansprüchen 1 bis 5, **dadurch gekennzeichnet, dass** das Glucomannan ein Molekulargewicht zwischen 1,9 x 10⁶ und 1 x 10⁴ hat.

7. Zusammensetzung zur Verwendung gemäß den Ansprüchen 1 bis 6, **dadurch gekennzeichnet, dass** das Glucomannan von einer natürlichen Quelle, vorzugsweise von der *Amorphophallus konjac-*Wurzel, abgeleitet ist.

8. Zusammensetzung zur Verwendung gemäß den Ansprüchen 1 bis 6, **dadurch gekennzeichnet, dass** das Glucomannan durch ein synthetisches Verfahren gewonnen wird.

9. Zusammensetzung zur Verwendung gemäß Anspruch 2, **dadurch gekennzeichnet, dass** die Carbonate aus der Gruppe gewählt sind, die aus basischem Magnesiumcarbonat und Calciumcarbonat besteht.

10. Zusammensetzung zur Verwendung gemäß einem beliebigen der obigen Ansprüche, **dadurch gekennzeichnet, dass** sie weiter, allein oder in Kombination, Folgendes umfasst: Alginate und ihre Derivate, Polyvinylalkohol, Poly(ethyloxazolin), Poly(2-hydroxyethylacrylat), Pektine, Gummiarabikum, Carboxymethylcellulose, Mischungen aus wasserlöslichen Polysacchariden, die abgeleitet sind von Pflanzenfasern, vermarktet unter dem Handelsnamen PGX®.

11. Zusammensetzung zur Verwendung gemäß einem beliebigen der obigen Ansprüche, **dadurch gekennzeichnet, dass** sie weiter Folgendes umfasst: pharmazeutisch verträgliche Bindemittel, wie zum Beispiel Lösungsmittel, Dispergiermittel, Träger, Phospholipide, Geschmacksstoffe und Farbstoffe.

12. Zusammensetzung zur Verwendung gemäß einem beliebigen der obigen Ansprüche, **dadurch gekennzeichnet, dass** sie zur oralen Verabreichung geeignet ist.

13. Zusammensetzung zur Verwendung gemäß Anspruch 12, **dadurch gekennzeichnet, dass** sie in fester Form vorliegt, als Pulver, Körnchen, Kapseln, Tabletten, in flüssiger Form, als Suspension, Lösung, Emulsion, Gel, Beutel zur Verabreichung als Rezeptur.

14. Zusammensetzung zur Verwendung gemäß beliebigen obigen Ansprüchen, **dadurch gekennzeichnet, dass** sie Folgendes umfasst: Chitosan, Glucomannan: 0,3 bis 10 Gewichtsteile Chitosan, vorzugsweise 3,5 Gewichtsteile Chitosan, basisches Magnesiumcarbonat: 0,1 bis 8 Gewichtsteile Chitosan, vorzugsweise 0,5 Gewichtsteile Chitosan, Calciumcarbonat: von 0,1 bis 8 Gewichtsteile Chitosan, vorzugsweise 0,5 Gewichtsteile Chitosan, Pulver von *Curcuma longa* und/oder Curcumin: von 0,1 bis 16 Gewichtsteile Chitosan, vorzugsweise 0,3 Gewichtsteile Chitosan.

15. Zusammensetzung zur Verwendung gemäß einem beliebigen der obigen Ansprüche, **dadurch gekennzeichnet, dass** sie Folgendes umfasst: Chitosan, Glucomannan: 3,5 Gewichtsteile Chitosan, basisches Magnesiumcarbonat: 0,5 Gewichtsteile Chitosan, Calciumcarbonat: 0,5 Gewichtsteile Chitosan, Pulver von *Curcuma longa* und/oder Curcumin: 0,3 Gewichtsteile Chitosan.

## Revendications

1. Composition pour utilisation dans la prévention et le traitement de troubles liés au reflux gastro-oesophagien et ses complications, comprenant une association, **caractérisée en ce qu'**elle comprend du chitosane, du glucomannane, et de la poudre de *Curcuma longa* et/ou de la curcumine, cette association étant le principe actif de la composition appropriée pour moduler le taux d'acidité du contenu gastrique qui est empêché de remonter dans l'oesophage.

2. Composition pour utilisation conforme à la revendication 1, **caractérisée en ce qu'**elle comprend en outre un ou plusieurs carbonate(s) afin de réguler le pH du contenu gastrique en deux étapes successives, dont la première est due à l'action des carbonates présents dans l'association et la deuxième à l'action du chitosane.

3. Composition pour utilisation conforme à la revendication 1 ou 2, **caractérisée en ce qu'**elle comprend l'association de polymères de chitosane et/ou de mélanges de tels polymères, et de polymères de glucomannane et/ou de mélanges de tels polymères.

4. Composition pour utilisation conforme à la revendication 1 ou 2, **caractérisée en ce que** le chitosane présente un pKb qui vaut de 6 à 10, de sorte qu'en présence d'ions H⁺, il donne des acides conjugués faibles dont le pKa vaut de 8 à 4.

5. Composition pour utilisation conforme à la revendication 1 ou 2, **caractérisée en ce que**, dans la molécule de glucomannane, le mannose et le glucose sont respectivement en un rapport de 1,6/1.

6. Composition pour utilisation conforme à l'une des revendications 1 à 5, **caractérisée en ce que** le glucomannane présente une masse molaire comprise entre 1,9.10⁶ et 1.10⁴.

7. Composition pour utilisation conforme à l'une des revendications 1 à 6, **caractérisée en ce que** le glucomannane provient d'une source naturelle, de préférence de racines d'*Amorphophallus konjac.*

8. Composition pour utilisation conforme à l'une des revendications 1 à 6, **caractérisée en ce que** le glucomannane est obtenu par voie de synthèse.

9. Composition pour utilisation conforme à la revendication 2, **caractérisée en ce que** les carbonates sont choisis dans l'ensemble formé par le carbonate basique de magnésium et le carbonate de calcium.

10. Composition pour utilisation conforme à l'une des revendications précédentes, **caractérisée en ce qu'**elle comprend en outre, seuls ou en combinaison, des alginates ou de leurs dérivés, du poly(alcool vinylique), de la poly(éthyl-oxazoline), du poly(acrylate de 2-hydroxy-éthyle), des pectines, de la gomme arabique, de la carboxyméthyl-cellulose, et/ou des mélanges de polysaccharides hydrosolubles dérivés de fibres végétales, commercialisés sous la marque PGX®.

11. Composition pour utilisation conforme à l'une des revendications précédentes, **caractérisée en ce qu'**elle comprend en outre des excipients pharmacologiquement admissibles, tels que solvants, dispersants, véhicule, phospholipides, arômes et colorants.

12. Composition pour utilisation conforme à l'une des revendications précédentes, **caractérisée en ce qu'**elle est appropriée pour être administrée par voie orale.

13. Composition pour utilisation conforme à la revendication 12, **caractérisée en ce qu'**elle se présente sous forme solide, telle que poudre, granules, gélules ou comprimés, sous forme liquide, telle que suspension, solution, émulsion ou gel, ou en sachets pour administration extemporanée.

14. Composition pour utilisation conforme à l'une des revendications précédentes, **caractérisée en ce qu'**elle comprend :
- chitosane,
- glucomannane : 0,3 à 10 parties en poids de chitosane, de préférence 3,5 parties en poids de chitosane ;
- carbonate basique de magnésium : 0,1 à 8 parties en poids de chitosane, de préférence 0,5 partie en poids de chitosane ;
- carbonate de calcium : 0,1 à 8 parties en poids de chitosane, de préférence 0,5 partie en poids de chitosane ;
- poudre de *Curcuma longa* et/ou curcumine : 0,1 à 16 parties en poids de chitosane, de préférence 0,3 partie en poids de chitosane.

15. Composition pour utilisation conforme à l'une des revendications précédentes, **caractérisée en ce qu'**elle comprend :
- chitosane,
- glucomannane : 3,5 parties en poids de chitosane ;
- carbonate basique de magnésium : 0,5 partie en poids de chitosane ;
- carbonate de calcium : 0,5 partie en poids de chitosane ;
- poudre de *Curcuma longa* et/ou curcumine : 0,3 partie en poids de chitosane.
